(19) Europäisches Patentamt
European Patent Office
Office européen des brevets

(11) Veröffentlichungsnummer: **0 006 992**
**A1**

(12) # EUROPÄISCHE PATENTANMELDUNG

(21) Anmeldenummer: 79101635.5

(22) Anmeldetag: 29.05.79

(51) Int. Cl.³: C 07 D 211/10
C 07 D 211/20, C 07 D 265/30
C 07 D 295/02, C 07 C 87/02
A 01 N 9/20, A 01 N 9/22

(30) Priorität: 14.06.78 DE 2825961

(43) Veröffentlichungstag der Anmeldung:
23.01.80 Patentblatt 80/2

(84) Benannte Vertragsstaaten:
AT BE CH DE FR GB IT LU NL SE

(71) Anmelder: BASF Aktiengesellschaft
Carl-Bosch-Strasse 38
D-6700 Ludwigshafen(DE)

(72) Erfinder: Himmele, Walter, Dr.
Eichenweg 14
D-6909 Walldorf(DE)

(72) Erfinder: Pommer, Ernst-Heinrich,Dr.
Berliner Platz 7
D-6703 Limburgerhof(DE)

(72) Erfinder: Goetz, Norbert, Dr.
Schoefferstrasse 25
D-6520 Worms(DE)

(54) **3-(p-Isopropyl-phenyl)-2-methyl-n-propyl-amine, diese enthaltende Fungizide, ein Verfahren zur Herstellung der genannten Verbindungen und Verfahren zur Bekämpfung von Pilzen unter Verwendung der genannten Verbindungen.**

(57) Die vorliegende Erfindung betrifft neue 3-(p-Isopropyl-phenyl)-2-methyl-n-propyl-amine, ein Verfahren zu ihrer Herstellung sowie Fungizide, die diese Amine, ihre Salze, Molekül- oder Additionsverbindungen als Wirkstoffe enthalten. Die Amine haben die Formel

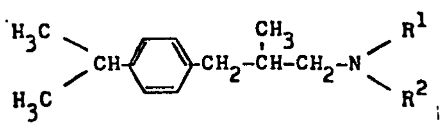

I

in der

$R^1$ Wasserstoff oder einen unverzweigten oder verzweigten Alkylrest mit 1 bis 4 Kohlenstoffatomen, einen unverzweigten oder verzweigten Alkoxyalkylrest mit 2 bis 6 Kohlenstoffatomen, einen gegebenenfalls einfach oder mehrfach durch unverzweigtes oder verzweigtes Alkyl mit 1 bis 4 Kohlenstoffatomen substituierten Cycloalkylrest mit 5 oder 6 Kohlenstoffatomen oder den Formylrest

und

$R^2$ Wasserstoff, einen unverzweigten oder verzweigten Alkylrest mit 1 bis 8 Kohlenstoffatomen, der gegebenenfalls durch Phenyl, das unverzweigte oder verzweigte Alkylgruppen mit 1 bis 4 Kohlenstoffatomen tragen kann, substituiert sein kann, einen unverzweigten oder verzweigten Alkoxyalkylrest mit 2 bis 6 Kohlenstoffatomen, einen Mono- oder Bicycloalkylrest mit 5 bis 9 Kohlenstoffatomen, der einfach oder mehrfach durch unverzweigtes oder verzweigtes Alkyl mit 1 bis 4 Kohlenstoffatomen substituiert sein kann, einen Indanyl-(1)-rest oder einen gegebenenfalls in 1-Stellung durch Methyl substituierten Piperidylrest bedeuten,

und

$R^1$ and $R^2$ zusammen mit dem Stickstoffatom, dessen Substituenten sie sind, einen gesättigten mono- oder bicyclischen Ring mit 5 bis 10 Ringgliedern, der ein weiteres Heteroatom als Ringlied enthalten und einfach oder mehrfach durch unverzweigtes oder verzweigtes Alkyl mit 1 bis 4 Kohlenstoffatomen, Hydroxyl, Hydroxylalkyl mit 1 bis 4 Kohlenstoffatomen, Alkoxy mit 1 bis 4 Kohlenstoffatomen, Pyridyl oder Benzyl substituiert sein kann, einen Pyrrolidonrest, einen Maleinimidrest, einen 1-Oxo-iso-indolinrest oder einen 1.2.3.4-Tetrahydrochinolinrest bilden.

EP 0 006 992 A1

0006992

BEZEICHNUNG GEÄNDERT
siehe Titelseite

BASF Aktiengesellschaft O. Z. 0050/033227

3-(p-Isopropyl-phenyl)-2-methyl-n-propyl-amine, ein Verfahren zu ihrer Herstellung und ihre Verwendung

Die vorliegende Erfindung betrifft neue 3-(p-Isopropyl-phenyl)-2-methyl-n-propyl-amine und ihre Salze, ein Verfahren zu ihrer Herstellung und Fungizide, die diese Amine als Wirkstoffe enthalten.

Es ist bekannt, daß 4-Alkyl-morpholinderivate und ihre Salze, beispielsweise N-Tridecyl-2,6-dimethylmorpholin, fungizid wirksam sind (DE-PS 1 164 152, DE-PS 1 173 722).

Es wurde gefunden, daß 3-(p-Isopropyl-phenyl)-2-methyl-n-propyl-amine der Formel I

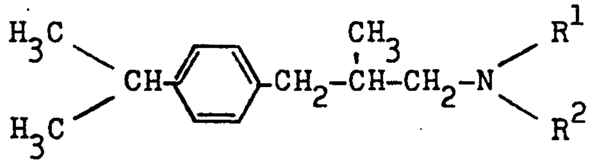

I

in der

$R^1$ Wasserstoff oder einen unverzweigten oder verzweigten Alkylrest mit 1 bis 4 Kohlenstoffatomen, einen unverzweigten oder verzweigten Alkoxyalkylrest mit 2 bis 6 Kohlenstoffatomen, einen gegebenenfalls einfach oder mehrfach durch unverzweigtes oder verzweigtes Alkyl mit 1 bis 4 Kohlenstoffatomen substituierten Cycloalkylrest mit 5 oder 6 Kohlenstoffatomen oder den Formylrest

H/ML

und

$R^2$ Wasserstoff, einen unverzweigten oder verzweigten Alkylrest mit 1 bis 8 Kohlenstoffatomen, der gegebenenfalls durch Phenyl, das unverzweigte oder verzweigte Alkylgruppen mit 1 bis 4 Kohlenstoffatomen tragen kann, substituiert sein kann, einen unverzweigten oder verzweigten Alkoxyalkylrest mit 2 bis 6 Kohlenstoffatomen, einen Mono- oder Bicycloalkylrest mit 5 bis 9 Kohlenstoffatomen, der einfach oder mehrfach durch unverzweigtes oder verzweigtes Alkyl mit 1 bis 4 Kohlenstoffatomen substituiert sein kann, einen Indanyl-(1)-rest oder einen gegebenenfalls in 1-Stellung durch Methyl substituierten Piperidylrest bedeuten,

und

$R^1$ und $R^2$ zusammen mit dem Stickstoffatom, dessen Substituenten sie sind, einen gesättigten mono- oder bicyclischen Ring mit 5 bis 10 Ringgliedern, der ein weiteres Heteroatom als Ringglied enthalten und einfach oder mehrfach durch unverzweigtes oder verzweigtes Alkyl mit 1 bis 4 Kohlenstoffatomen, Hydroxyl, Hydroxyalkyl mit 1 bis 4 Kohlenstoffatomen, Alkoxy mit 1 bis 4 Kohlenstoffatomen, Pyridyl oder Benzyl substituiert sein kann, einen Pyrrolidonrest, einen Maleinimidrest, einen 1-Oxo-iso-indolinrest oder einem 1.2.3.4-Tetrahydrochinolinrest bilden, oder ihre Salze, Molekül- und Additionsverbindungen, eine sehr gute fungizide Wirkung haben und sich zur Bekämpfung von Pflanzenkrankheiten eignen.

In der Formel I steht $R^1$ für Wasserstoff, für unverzweigte oder verzweigte Alkylreste mit 1 bis 4 Kohlenstoffatomen, beispielsweise Methyl, Äthyl, Isopropyl, n-Butyl, i-Butyl, für unverzweigte oder verzweigte Alkoxyalkylreste mit 2 bis 6 Kohlenstoffatomen, beispielsweise Methoxymethyl, 2-Methoxyäthyl, 1-Methyl-2-methoxyäthyl, 1-Methyl-2-propoxyäthyl, oder für einen Cycloalkylrest mit 5 oder 6 Koh-

lenstoffatomen, der durch Alkylreste mit 1 bis 4 Kohlenstoffatomen, beispielsweise Methyl, Äthyl, Isopropyl, tert.-Butyl, vorzugsweise Methyl, einfach oder mehrfach substituiert sein kann.

$R^2$ in Formel I bedeutet einen unverzweigten oder verzweigten Alkylrest mit 1 bis 8 Kohlenstoffatomen, beispielsweise Methyl, Äthyl, Isopropyl, n-Propyl, n-Butyl, i-Butyl, sec.-Butyl, n-Hexyl, Isohexyl, n-Octyl, 1,1,3,3-Tetramethyl-n-hexyl. Diese Alkylreste können durch Phenyl, das Alkylreste mit 1 bis 4 Kohlenstoffatomen, beispielsweise Methyl, Äthyl, Isopropyl, tert.-Butyl, tragen kann, substituiert sein. Beispiele für diese phenylsubstituierten Alkylreste sind Benzyl, 1-Phenyläthyl, 2-Methyl-2-phenyl-äthyl, 3-Methyl-3-phenyl-n-propyl, 3-Methyl-3-(4-isopropylphenyl)-n-propyl.

Als Alkoxyalkylreste für $R^2$ mit 2 bis 6 Kohlenstoffatomen kommen beispielsweise Methoxymethyl, 2-Methoxyäthyl, 2-Äthoxyäthyl, 1-Methyl-2-methoxyäthyl, 1-Methyl-2-propoxyäthyl, n-Butoxyäthyl, in Betracht.

$R^2$ kann darüber hinaus für einen Mono- oder Bicycloalkylrest mit 5 bis 9 Kohlenstoffatomen, beispielsweise für Cyclopentyl, Cyclohexyl, Cycloheptyl, Bicycloheptyl, Bicyclononyl, wie Bicyclo[3.1.1]-heptyl-(3), Bicyclo[4.3.0]-nonyl-(7), stehen, der durch Alkylreste mit 1 bis 4 Kohlenstoffatomen, beispielsweise Methyl, Äthyl, Isopropyl, tert.-Butyl, Isobutyl, einfach oder mehrfach substituiert sein kann. Bevorzugt sind der Cyclohexylrest oder alkylsubstituierte Cyclohexylreste. Weitere cyclische Reste, die für $R^2$ stehen können, sind der Indanyl-(1)-rest oder der Piperidylrest, der in 1-Stellung durch Methyl substituiert sein kann.

$R^1$ und $R^2$ können zusammen mit den Stickstoffatomen, dessen Substituenten sie sind, gesättigte mono- oder bicyclische Ringe mit 5 bis 10 Ringgliedern bilden, die noch ein weiteres Heteroatom, beispielsweise ein Stickstoff- oder ein Sauerstoffatom, als Ringglied enthalten können. Beispiele für diese Ringe sind Pyrrolidinyl, Piperidyl, Morpholinyl, Piperazinyl, Azacycloheptyl, Dekahydrochinolyl, Dekahydroisochinolin, Oktahydroindolyl, 6-Azabicyclo[3.2.1]-octyl und Isoindolinyl. Diese Ringe können einfach oder mehrfach durch Alkyl mit 1 bis 4 Kohlenstoffatomen, insbesondere Methyl, Äthyl oder tert. Butyl, durch Hydroxyl, Hydroxylalkyl mit 1 bis 4 Kohlenstoffatomen, insbesondere Hydroxymethyl oder 2-Hydroxyäthyl, Alkoxy mit 1 bis 4 Kohlenstoffatomen, insbesondere Methoxy, Pyridylreste, insbesondere den Pyridyl-(2)-rest, oder Benzyl substituiert sein.

$R^1$ und $R^2$ können außerdem zusammen mit dem Stickstoffatom, dessen Substituenten sie sind, einen Pyrrolidonrest, einen Maleinimidrest, einen 1-Oxo-isoindolinrest oder einen 1.2.3.4-Tetrahydrochinolinrest bilden.

Bevorzugte Amine der Formel I sind solche, bei denen $R^1$ und $R^2$ zusammen mit dem Stickstoffatom, dessen Substituenten sie sind, Morpholinyl, oder durch Alkyl substituiertes Morpholinyl, wie 2,6-Dimethyl-morpholinyl, 2,6-Diäthyl-morpholinyl oder 3,5-Dimethyl-morpholinyl, Piperidyl oder durch Alkyl substituiertes Piperidyl, wie 3,5-Dimethyl--piperidyl, 3-Methyl-piperidyl, 4-Äthyl-piperidyl, 3,4-Dimethyl-piperidyl, 3-Äthyl-piperidyl oder 3,5-Diäthyl-piperidyl, bedeuten.

Als Salze der Amine der Formel I kommen Salze mit anorganischen Säuren, beispielsweise Chlorwasserstoffsäure, Bromwasserstoffsäure, Jodwasserstoffsäure, Schwefelsäure, Phosphorsäure, Salpetersäure, Fluorwasserstoffsäure, oder mit

organischen Säuren, beispielsweise Essigsäure, Propionsäure, Ameisensäure, Fumarsäure, Adipinsäure, Weinsäure, Benzoesäure, Salicylsäure, 3,5-Dichlorsalicylsäure, in Betracht. Molekül- und Additionsverbindungen entstehen beispielsweise mit Säuren von Tensiden, wie mit Dodecylbenzolsulfonsäure.

Amine der Formel I können durch Umsetzung von 3-(p-Isopropyl-phenyl)-2-methyl-propanal mit einem Amin der Formel II

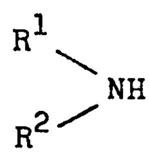

II,

in der
$R^1$ und $R^2$ die oben genannten Bedeutungen haben,
in Gegenwart eines Reduktionsmittels bei einer Temperatur im Bereich von 20 bis 250°C erhalten werden.

Als Reduktionsmittel eignen sich Ameisensäure, Oxalsäure, Wasserstoff in Gegenwart eines Katalysators sowie Wasserstoff-liefernde Verbindungen wie $N_2H_x$, vorzugsweise Ameisensäure. Das Reduktionsmittel wird in Mengen von 1 bis 5 Mol, bezogen auf 1 Mol Ausgangssubstanz, eingesetzt.

Die Umsetzung wird bei einer Temperatur im Bereich von 20 bis 250°C durchgeführt. Wird Ameisensäure als Reduktionsmittel verwendet, arbeitet man bei einer Temperatur zwischen 80 und 160°C, vorzugsweise zwischen 120 und 140°C.

Die Ausgangsverbindungen sind bekannt und können nach an sich bekannten Methoden hergestellt werden.

Die folgenden Beispiele erläutern die Herstellung der neuen Verbindungen.

Beispiel 1

127 g 3-(p-Isopropyl-phenyl)-2-methyl-propanal werden unter Rühren mit 57 g Piperidin versetzt. Im Verlauf einer Stunde werden zu diesem Gemisch 34 g Ameisensäure zugegeben. Das Reaktionsgemisch wird anschließend 12 Stunden auf 130°C erwärmt; es entweicht $CO_2$.

Nach Beendigung der reduktiven Aminierung wird das Amin durch fraktionierte Destillation gereinigt. Der Vorlauf geht bis zu einer Temperatur von 156°C bei 4 mbar über (21 g). Der Hauptlauf (153 g) geht bei dem gleichen Druck zwischen 156 bis 158°C über. Als Nachlauf werden bis zu einer Übergangstemperatur von 166°C 5 g erhalten. Fraktion 2 und Fraktion 3 bestehen laut gaschromatographischer Analyse aus reinem 1-[3'-(p-Isopropylphenyl)-2'-methyl-n-propyl]-piperidin.

Analysen:

$C_{18}H_{29}N_1$

| | C | H | N |
|---|---|---|---|
| ber. | 83,33 % | 11,17 % | 5,40 % |
| gef. | 83,3 % | 11,0 % | 5,5 % |

Das NMR-Spektrum zeigt die für die Verbindung zu erwartenden Signale.

Beispiel 2

190 g 3-(p-Isopropyl-phenyl)-2-methyl-propanal werden mit 50 g Ameisensäure versetzt. In diese Mischung tropft man im Verlauf von einer Stunde 73 g N-Methyl-isopropylamin.

Das Reaktionsgemisch erwärmt sich. Zur Vervollständigung der Umsetzung wird noch 12 Stunden auf etwa 120°C erhitzt. Dabei findet $CO_2$-Abspaltung statt.

Die Isolierung und Reinigung des 3-(p-Isopropylphenyl)-2-methyl-1-(N-methyl-N-isopropylamino)-n-propans erfolgt durch Fraktionierung unter einem Druck von 4 mbar. Als Vorlauf werden 19 g erhalten. Dieser geht bis zu einer Temperatur von 108°C über. Der Hauptlauf (213 g) geht zwischen 108 und 112°C über. Nachlauf bis zu einer Übergangstemperatur von 122°: 6 g. Destillationsrückstand: 6 g.

Die gaschromatographische Analyse zeigt, daß der Vorlauf zu über 80 % aus 3-(p-Isopropylphenyl)-2-methyl-1-(N-methyl-N-isopropyl-amino)-n-propan besteht. Hauptlauf und Nachlauf bestehen zu mehr als 90 % aus diesem Produkt. Die Ausbeute beträgt 95 %, bezogen auf eingesetzten Aldehyd.

Beispiel 3

Zu 100 g Essigester, der bei 20°C mit trockenem Chlorwasserstoff gesättigt wurde, werden unter Kühlung mit Eis tropfenweise 50 g 3-(p-Isopropylphenyl)-2-methyl-1-(N-methyl-N-isopropyl-amino)-n-propan zugegeben. Das Hydrochlorid des Amins fällt in gut isolierbaren Kristallen aus. Durch Absaugen und Trocknen werden 45 g gewonnen; Fp. 134°C.

Beispiel 4

Wie im Beispiel 1 beschrieben, werden 127 g 3-(p-Isopropylphenyl)-2-methyl-propanal mit 67 g N-Methyl-piperazin und 34 g Ameisensäure umgesetzt. Durch Fraktionierung werden bei einem Druck von 6,5 mbar zwischen 160 und 165°C 148 g des entsprechenden Piperazinderivates erhalten. Die Rein-

heit beträgt laut gaschromatographischer Analyse mehr als 97 %.

Werden 100 g der so hergestellten Verbindung in 300 g Methanol gelöst und in 200 g methanolische Chlorwasserstofflösung gegeben, so kristallisiert sofort das Bishydrochlorid des 1-[3'-(p-Isopropylphenyl)-2'-methyl-n-propyl]-4-methyl-piperazins aus; Fp. 240°C.

Analog lassen sich folgende Verbindungen herstellen:

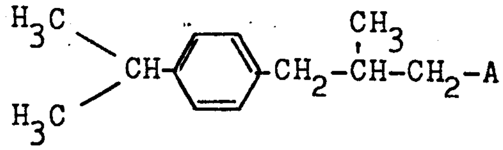

| Nr. | A = 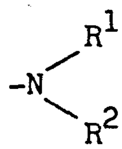 | Kp der Base bzw. Fp des Salzes [°C] |
|---|---|---|
| 1 | 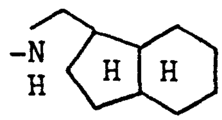 | $Kp_5$ : 200 - 212 |
| 2 | 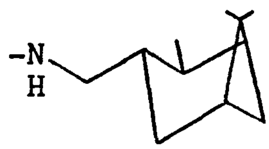 | $Kp_5$ : 225 - 235 |
| 3 | 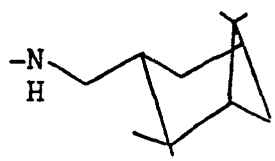 | $Kp_5$ : 227 - 240 |
| 4 | 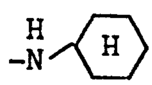 | $Kp_{0,3}$: 120 - 130 |
| 5 | 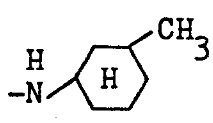 | $Kp_5$ : 142 - 152 |
| 6 | 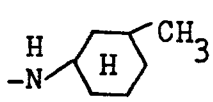 | $Kp_5$ : 153 - 158 |
| 7 | 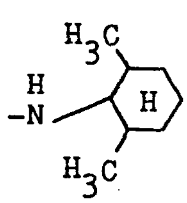 | $Kp_5$ : 165 - 178 |

| Nr. | A = 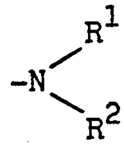 | Kp der Base bzw. Fp des Salzes [°C] |
|---|---|---|
| 8 | 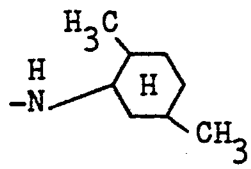 | $Kp_5$ : 175 - 195 |
| 9 | 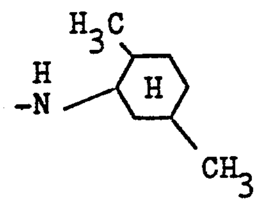 | $Kp_5$ : 185 - 195 |
| 10 | 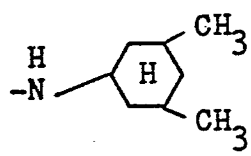 | $Kp_5$ : 175 - 185 |
| 11 | 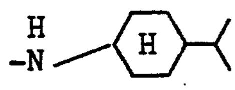 | $Kp_5$ : 190 - 197 |
| 12 | 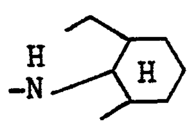 | $Kp_5$ : 193 - 195 |
| 13 | 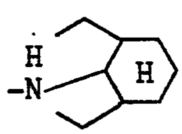 | $Kp_3$ : 147 - 153 |

| Nr. | 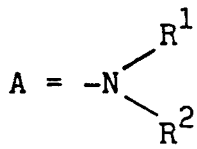 | Kp der Base bzw. Fp des Salzes [°C] |
|---|---|---|
| 14 | $NH_2$ | $Kp_{20}$ : 148 - 150 |
| 15 | $NH_2$ . HCl | Fp : 198 |
| 16 | $-NH(CH_3)$ | $Kp_{10}$ : 126 |
| 17 | $-NH(CH_3)$ . HCl | Fp : 117 |
| 18 | 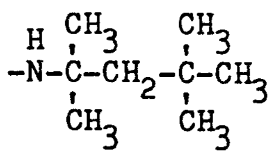 | $Kp_5$ : 166 - 170 |
| 19 | 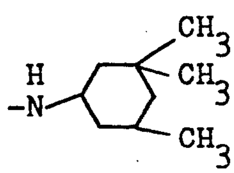 | $Kp_{0,3}$: 138 - 140 |
| 20 | 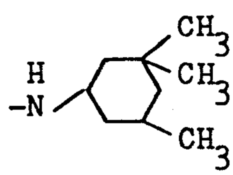 . HCl | Fp : 160 |
| 21 | 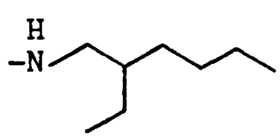 | $Kp_5$ : 202 - 210 |
| 22 | 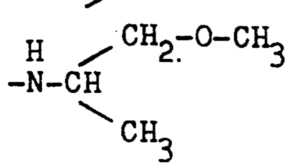 | $Kp_{0,2}$: 170 - 180 |

| Nr. | 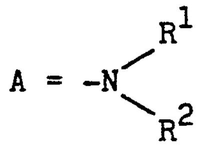 | | Kp der Base bzw. Fp des Salzes [°C] |
|---|---|---|---|
| 23 | 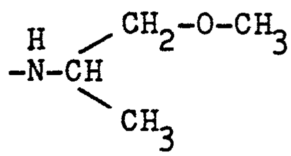 | . HCl | Fp : 103 |
| 24 | 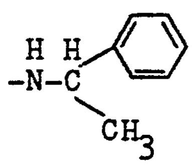 | | $Kp_{0,3}$: 148 - 160 |
| 25 | 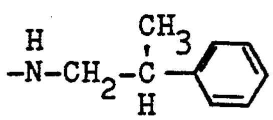 | | $Kp_5$ : 210 - 220 |
| 26 | 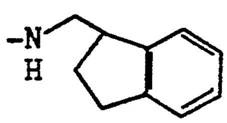 | | $Kp_5$ : 210 - 230 |
| 27 | 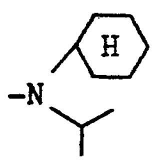 | | $Kp_5$ : 170 - 176 |
| 28 | 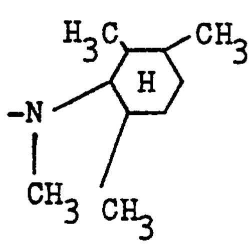 | | $Kp_5$ : 190 - 210 |
| 29 | 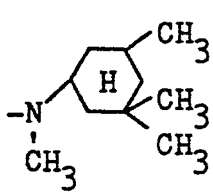 | | $Kp_3$ : 145 - 155 |

| Nr. | A = 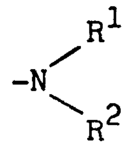 | Kp der Base bzw. Fp des Salzes [°C] |
|---|---|---|
| 30 | 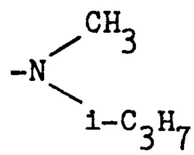 | $Kp_{30}$ : 165 - 175 |
| 31 | 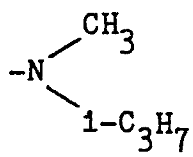 · HCl | Fp : 134 |
| 32 | $-N(i-C_3H_7)_2$ | $Kp_5$ : 138 - 140 |
| 33 | $-N(i-C_3H_7)_2$ · HCl | Fp : 122 |
| 34 | 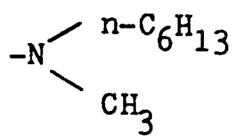 | $Kp_5$ : 145 - 150 |
| 35 | $-N(n-C_4H_9)_2$ | $Kp_{0,3}$: 132 - 135 |
| 36 | 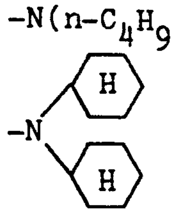 | $Kp_5$ : 200 - 205 |
| 37 | $-N(CH_3)_2$ | $Kp_{12}$ : 140 - 141 |
| 38 | $-N(CH_3)_2$ · HCl | Fp : 204 |
| 39 | $-N(C_2H_5)_2$ | $Kp_5$ : 136 - 139 |
| 40 | $-N(i-C_4H_9)_2$ | $Kp_5$ : 158 - 160 |

| Nr. | 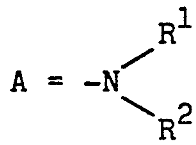 | Kp der Base bzw. Fp des Salzes [°C] |
|---|---|---|
| 41 | 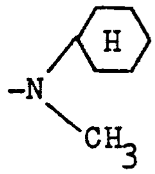 | $Kp_3$ : 163 - 168 |
| 42 | 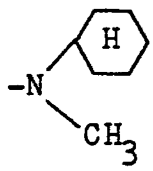 . HCl | Fp : 196 |
| 43 | 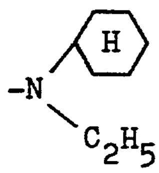 | $Kp_{0,3}$: 140 - 142 |
| 44 | 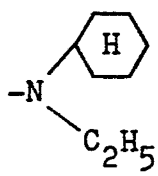 . HCl | Fp : 155 |
| 45 | 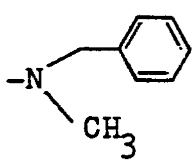 | $Kp_5$ : 176 - 180 |
| 46 | 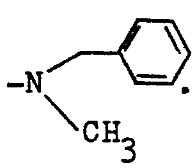 . HCl | Fp : 158 |
| 47 | 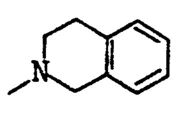 | $Kp_{0,2}$: 160 - 178 |
| 48 | 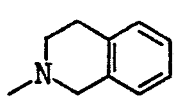 . HCl | Fp : 218 |

| Nr. | A = 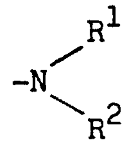 | Kp der Base bzw. Fp des Salzes [°C] |
|---|---|---|
| 49 | 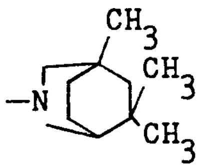 | $Kp_1$ : 140 - 145 |
| 50 | 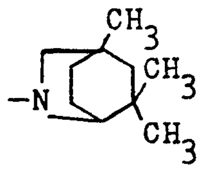 . HCl | (nicht kristallin) |
| 51 | 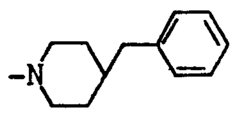 | $Kp_3$ : 190 - 198 |
| 52 | 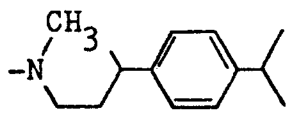 | $Kp_{0,5}$: 182 - 185 |
| 53 | 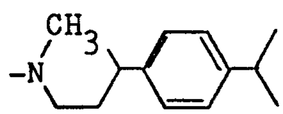 . HCl | Fp : 184 |
| 54 | 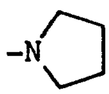 | $Kp_5$ : 140 - 145 |
| 55 | 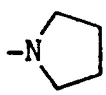 . HCl | Fp : 176 |
| 56 | 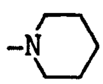 | $Kp_5$ : 150 - 152 |
| 57 | 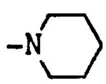 . HCl | Fp : 212 |
| 58 | 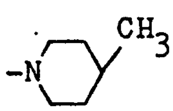 | $Kp_5$ : 157 - 162 |

| Nr. | A = 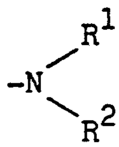 | Kp der Base bzw. Fp des Salzes [°C] |
|---|---|---|
| 59 | 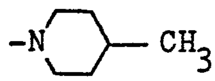 . HCl | Fp : 222 |
| 60 | 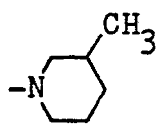 | $Kp_{0,3}$: 110 - 115 |
| 61 | 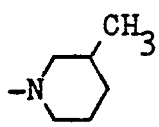 . HCl | Fp : 166 |
| 62 | 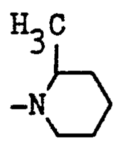 | $Kp_{0,3}$: 107 - 112 |
| 63 | 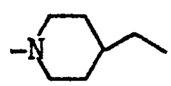 | $Kp_{0,3}$: 145 - 150 |
| 64 | 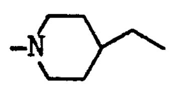 . HCl | Fp : 230 |
| 65 | 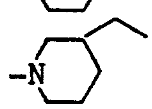 | $Kp_5$ : 165 - 170 |
| 66 | 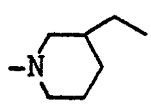 . HCl | Fp : 154 |
| 67 | 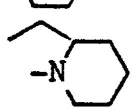 | $Kp_5$ : 160 - 165 |
| 68 | 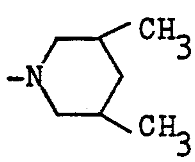 | $Kp_5$ : 158 - 164 |

| Nr. | A = 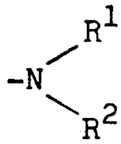 | Kp der Base bzw. Fp des Salzes [°C] |
|---|---|---|
| 69 | 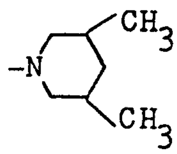 · HCl | Fp : 184 |
| 70 | 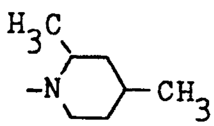 | $Kp_{0,2}$: 120 - 125 |
| 71 | 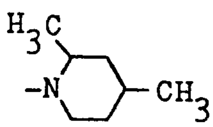 · HCl | Fp : 156 |
| 72 | 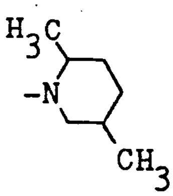 | $Kp_8$ : 180 - 185 |
| 73 | 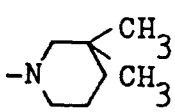 | $Kp_5$ : 160 - 165 |
| 74 | 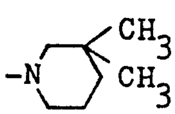 · HCl | Fp : 182 |
| 75 | 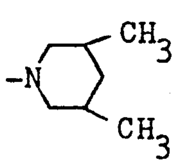 (cis-Form) | $Kp_5$ : 153 - 160 |
| 76 | 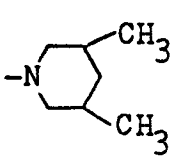 · HCl (cis-Form) | Fp : 202 |

| Nr. | A = 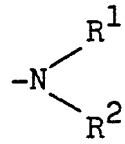 | Kp der Base bzw. Fp des Salzes [°C] |
|---|---|---|
| 77 | 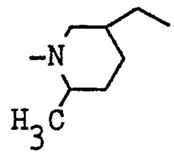 | $Kp_5$ : 170 - 173 |
| 78 | 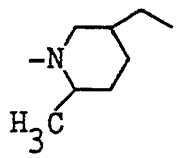 . HCl | Fp : 140 |
| 79 | 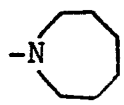 | $Kp_5$ : 165 - 168 |
| 80 | 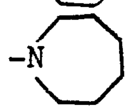 . HCl | Fp : 207 |
| 81 | 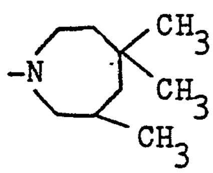 | $Kp_3$ : 140 - 156 |
| 82 | 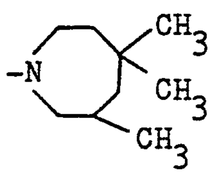 . HCl | Fp : 176 |
| 83 | 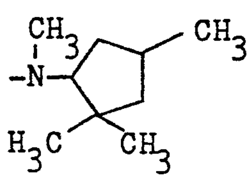 | $Kp_5$ : 198 - 212 |
| 84 | 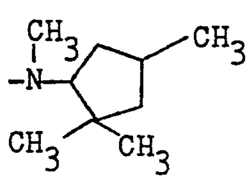 . HCl | Fp : 178 |

| Nr. | 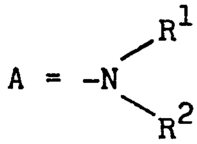 | Kp der Base bzw. Fp des Salzes [°C] |
|---|---|---|
| 85 | 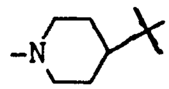 | $Kp_5$ : 190 - 197 |
| 86 | 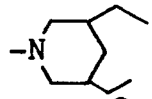 | $Kp_5$ : 180 - 182 |
| 87 | 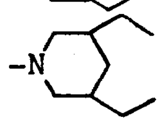 . HJ | Fp : 145 |
| 88 | 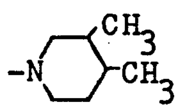 | $Kp_5$ : 150 - 155 |
| 89 | 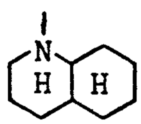 | $Kp_3$ : 155 - 165 |
| 90 | 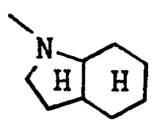 | $Kp_9$ : 210 - 217 |
| 91 | 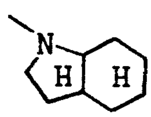 . HCl | Fp : 210 |
| 92 | 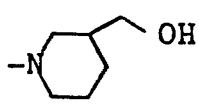 | $Kp_5$ : 205 - 210 |

| Nr. | A = 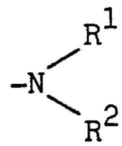 | Kp der Base bzw. Fp des Salzes [°C] |
|---|---|---|
| 93 | 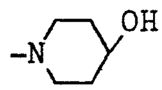 | $Kp_5$ : 175 - 185 |
| 94 | 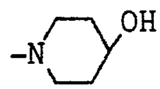 | Fp : 143 |
| 95 | 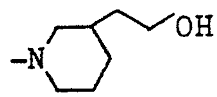 | $Kp_{0,2}$: 156 - 160 |
| 96 | 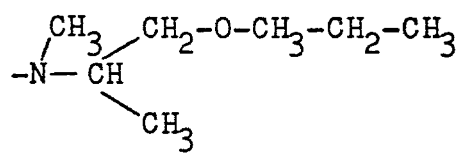 | $Kp_{0,2}$: 126 - 130 |
| 97 | 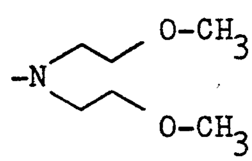 | $Kp_5$ : 172 - 177 |
| 98 | 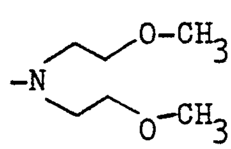 . HCl | |
| 99 | 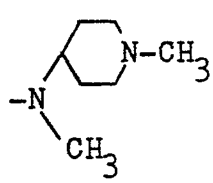 | $Kp_{0,2}$: 145 - 150 |
| 100 | 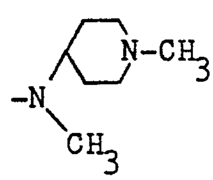 . 2 HCl | Fp : 235 |

| Nr. | A = 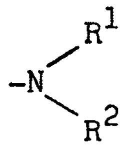 | Kp der Base bzw. Fp des Salzes [°C] |
|---|---|---|
| 101 | 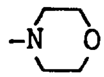 | $Kp_5$ : 170 - 172 |
| 102 | 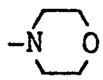 . HCl | Fp : 214 |
| 103 | 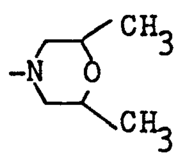 (trans-Form) | $Kp_{0,3}$: 120 - 130 |
| 104 | 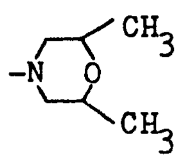 . HCl (trans-Form) | Fp : 164 |
| 105 | 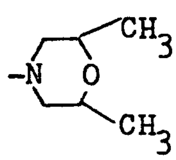 (cis-Form) | $Kp_5$ : 160 - 164 |
| 106 | 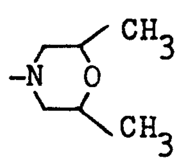 . HCl (cis-Form) | Fp : 193 |
| 107 | 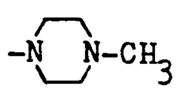 | $Kp_5$ : 160 - 165 |
| 108 | 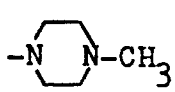 . 2 HCl | Fp : 240 |
| 109 | 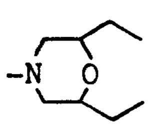 | $Kp_5$ : 190 - 200 |
| 110 | 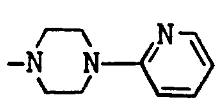 | $Kp_5$ : 245 - 250 |

| Nr. | A = 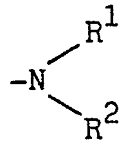 | Kp der Base bzw. Fp des Salzes [°C] |
|---|---|---|
| 111 | 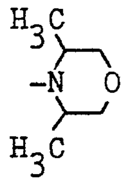 | $Kp_5$ : 130 - 140 |
| 112 | 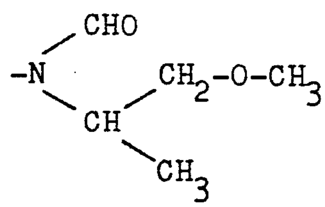 | $Kp_5$ : 198 - 215 |
| 113 | 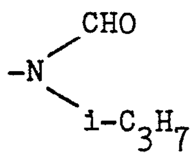 | $Kp_5$ : 193 - 195 |
| 114 | 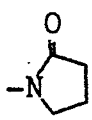 | $Kp_5$ : 19 |
| 115 | 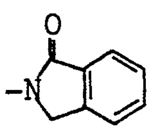 | $Kp_5$ : 240 - 250 |
| 116 | 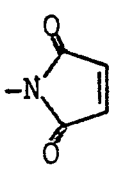 | $Kp_{0,3}$ : 156 - 160 |

Die erfindungsgemäßen Wirkstoffe und die entsprechenden Fungizide sind insbesondere geeignet zur Bekämpfung von Pflanzenkrankheiten, z.B. Erysiphe graminis (echter Mehltau) an Getreide, Erysiphe cichoriacearum (echter Mehltau) an Kürbisgewächsen, Podosphaera leucotricha an Äpfeln, Uncinula necator an Reben, Erysiphe polygoni an Bohnen, Sphaerotheca pannosa an Rosen, Microsphaera querci an Eichen, Botrytis cinerea an Erdbeeren, Reben, Mycophaerella musicola an Bananen, Puccinia-Arten (Rostpilze) an Getreide, Uromyces appendiculatus und Uromyces phaseoli an Bohnen, Hemileia vastatrix an Kaffee und Rhizoctonia solani. Sie sind systemisch wirksam; sie werden sowohl über die Wurzeln als auch über die Blätter aufgenommen und im Pflanzengewebe transportiert.

Bei der Anwendung der neuen Wirkstoffe zur Behandlung von Pflanzen gegen Pilzinfektionen liegen die Aufwandmengen zwischen 0,025 und 5 kg Wirkstoff/ha Fläche. Zum Oberflächenschutz von Baumen oder Früchten können die Wirkstoffe auch in Verbindung mit Kunststoffdispersionen 0,25 %-ig bis 5 %-ig, bezogen auf das Gewicht der Dispersion, verwendet werden. Die Fungizide enthalten im allgemeinen zwischen 0,1 und 95 Gew.%, vorzugsweise zwischen 0,5 und 90 %, Wirkstoff.

Die Wirkstoffe können mit anderen bekannten Fungiziden gemischt werden. In vielen Fällen erhält man dabei eine Vergrößerung des fungiziden Wirkungsspektrums; bei einer Anzahl von Fungizidmischungen in den Gewichtsverhältnissen 1 : 10 bis 10 : 1 treten auch synergistische Effekte auf, d.h. die fungizide Wirksamkeit des Kombinationsproduktes ist größer als die der addierten Wirksamkeiten der Einzelkomponenten. Fungizide, die mit den erfindungsgemäßen Verbindungen kombiniert werden können, sind beispielsweise:

Dithiocarbamate und deren Derivate, wie
Zinkdimethyldithiocarbamat,
Manganäthylenbisdithiocarbamat,
Mangan-Zink-äthylendiamin-bis-dithiocarbamat,
Zinkäthylenbisdithiocarbamat,
Tetramethylthiuramdisulfid,
Ammoniak-Komplex von Zink-(N,N-äthylen-bis-dithiocarbamat) und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;
heterocyclische Verbindungen, wie
N-Trichlormethylthio-tetrahydrophthalimid,
N-Trichlormethylthio-phthalimid,
N-(1,1,2,2-Tetrachloräthylthio)-tetrahydrophthalimid,
1-(Butylcarbamoyl)-2-benzimidazol-carbaminsäuremethylester,
2-Methyloxycarbonylamino-benzimidazol,
2,3-Dihydro-5-carboxanilido-6-methyl-1,4-oxathiin-4,4-dioxid,
2,3-Dihydro-5-carboxanilido-6-methyl-1,4-oxathiin,
5-Butyl-2-dimethylamino-4-hydroxy-6-methyl-pyrimidin,
1,2-Bis-(3-äthoxycarbonyl-2-thioureido)-benzol,
1,2-Bis-(3-methoxycarbonyl-2-thiorueido)-benzol
und verschiedene andere Fungizide, wie
Dededylguanidinacetat,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Jodbenzoesäureanilid,
2-Brombenzoesäureanilid,

3-Nitro-isophthalsäure-diisopropylester,
1-(1,2,4-Triazolyl-1')- 1-(4'-chlorphenoxy) -3,3-dimethylbutan-2-on,
1-(1-Imidazolyl)-2-allyloxy-2-(2,4-dichlorphenyl)-äthan,
Piperazin-1,4-diyl-bis-1-(2,2,2-Trichloräthyl)-formamid,
2,4,5,6-Tetrachlor-isophthalonitril,
1,2-Dimethyl-3,5-diphenyl-pyrazoliniummethylsulfat.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstel-

lung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und evtl. Lösungsmittel oder Öl bestehende Konzentrate hergestellt werden, die zur Verdünnung mit Wasser geeignet sind.

Als oberflächenaktiven Stoffe kommen Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyäthylen-octylphenoläther, äthoxyliertes Isooctylphenol-, Octylphenol-, Nonylphenol, Alkylphenolglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäure, Kieselgele, Silikate, Talkum, Kaolin, Attaclay, Kalkstein, Kalk, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden. Im allgemeinen liegen sie zwischen 0,001 und 10 %, vorzugsweise zwischen 0,01 und 1 %.

Zu den Mischungen oder Einzelwirkstoffen können Öle verschiedenen Typs, Herbizide, andere Fungizide, Nematozide, Insektizide, Bakterizide, Spurenelemente, Düngemittel, Antischaummittel (z.B. Silikone), Wachstumsregulatoren, Antidotmittel oder andere wirksame Verbindungen, zugesetzt werden.

Die folgenden Beispiele erläutern die Formulierung der Wirkstoffe:

<u>Beispiel 5</u>

Man vermischt 90 Gewichtsteile 1-[3'-(p-Isopropylphenyl)--2'-methyl-n-propyl]-piperidin mit 10 Gewichtsteilen N-Methyl- -pyrrolidon und erhält eine Lösung, die zur Anwendung in Form kleinster Tropfen geeignet ist.

Beispiel 6

20 Gewichtsteile 3-(p-Isopropylphenyl)-2-methyl-1-(N-methyl-N-isopropylamino)-n-propan werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-mono-äthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

Beispiel 7

20 Gewichtsteile 1-[3'-(p-Isopropylphenyl)-2'-methyl-n-propyl]-4-methyl-piperazin werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Beispiel 8

20 Gewichtsteile 1-[3'-(p-Isopropylphenyl)-2'-methyl-n-propyl]-4,4,6-trimethyl-1-azacycloheptan werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in

100 000 Gewichtsteilen Wasser erhält man eine wäßrige Dispersion, die 0,02 Gew.% des Wirkstoffs enthält.

Beispiel 9

20 Gewichtsteile 4-[3'-(p-Isopropylphenyl)-2'-methyl-n--propyl]-3,5-dimethyl-morpholin werden mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

Beispiel 10

3 Gewichtsteile 3-(p-Isopropylphenyl)-2-methyl-1-(N-2,6-dimethyl-cyclohexyl-amino)-n-propan werden mit 97 Gewichtsteilen feinteiligem Kaolin innig vermischt. Man erhält auf diese Weise ein Stäubemittel, das 3 Gew.% des Wirkstoffs enthält.

Beispiel 11

30 Gewichtsteile 4-[3'-(p-Isopropylphenyl)-2'-methyl-n--propyl]-2,6-dimethyl-morpholin werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 12

40 Gewichtsteile 4-[3'-(p-Isopropylphenyl)-2'-methyl-n--propyl]-2,6-dimethyl-morpholin-hydrochlorid werden mit 10 Teilen Natriumsalz eines Phenolsulfonsäure-harnstoff--formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

Beispiel 13

20 Teile 1-[3'-(p-Isopropylphenyl)-2'-methyl-n-propyl]--1.2.3.4-tetrahydrochinolin werden mit 2 Teilen Calciumsalz der Dodecylbenzolsulfonsäure, 8 Teile Fettalkohol-polyglykoläther, 2 Teile Natriumsalz eines Phenolsulfonsäure-harnstoff-formaldehyd-kondensats und 68 Teile eines paraffinischen Mineralöls innig vermischt. Man erhält eine stabile ölige Dispersion.

Für die biologischen Versuche wurden folgende bekannte Vergleichssubstanzen verwendet:

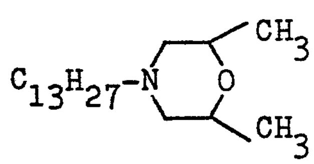 A

(DE-PS 1 164 152)

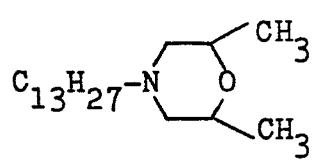 $\cdot$ $CH_3COOH$ B

(DE-PS 1 173 722)

Beispiel A

Blätter von in Töpfen gewachsenen Weizenkeimlingen der Sorte "Jubilar" werden mit wäßrigen Emulsionen aus 80 Gew.% Wirkstoff und 20 Gew.% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

| Wirkstoff-Nr. | Befall der Blätter nach Spritzungen mit x %-iger Wirkstoffbrühe x = 0,012 | 0,025 | 0,05 |
|---|---|---|---|
| 30 | 0 | 0 | 0 |
| 31 | 0 | 0 | 0 |
| 32 | 3 | 0 | 0 |
| 33 | 2 | 0 | 0 |
| 56 | 0 | 0 | 0 |
| 57 | 0 | 0 | 0 |
| 58 | 0 | 0 | 0 |
| 60 | 2 | 0 | 0 |
| 61 | 0 | 0 | 0 |
| 62 | 0 | 0 | 0 |
| 64 | 2 | 0 | 0 |
| 88 | 0 | 0 | 0 |
| 105 | 1 | 0 | 0 |
| 106 | 2 | 0 | 0 |
| 108 | 0 | 0 | 0 |
| A (bekannt) | 3 | 2 | 1 |
| B (bekannt) | 4 | 2 | 1 |
| Kontrolle (unbehandelt) | | 4 | |

0 = kein Befall, abgestuft bis 5 = Totalbefall

Beispiel B

In einem weiteren Versuch werden, wie in Beispiel A beschrieben, Blätter von in Töpfen gewachsenen Gerstenkeimlingen der Sorte "Firlbecks Union" behandelt und mit Sporen (Konidien) des Gerstenmehltaus (Erysiphe graminis var. hordei) bestäubt.

| Wirkstoff-Nr. | Befall der Blätter nach Spritzungen mit x %-iger Wirkstoffbrühe x = 0,012 | 0,025 |
|---|---|---|
| 7 | 0 | 0 |
| 14 | 0 | 0 |
| 30 | 0 | 0 |
| 31 | 0 | 0 |
| 32 | 0 | 0 |
| 33 | 0 | 0 |
| 47 | 0 | 0 |
| 55 | 1 | 0 |
| 56 | 0 | 0 |
| 57 | 0 | 0 |
| 58 | 0 | 0 |
| 59 | 1 | 0 |
| 60 | 0 | 0 |
| 61 | 0 | 0 |
| 62 | 0 | 0 |
| 66 | 0 | 0 |
| 75 | 0 | 0 |
| 76 | 0 | 0 |
| 81 | 0 | 0 |
| 82 | 0 | 0 |
| 85 | 0 | 0 |
| 88 | 0 | 0 |

| Wirkstoff-Nr. | Befall der Blätter nach Spritzungen mit x %-iger Wirkstoffbrühe x = 0,012 | 0,025 |
|---|---|---|
| 105 | 0 | 0 |
| 106 | 0 | 0 |
| 108 | 0 | 0 |
| 111 | 0 | 0 |
| A (bekannt) | 1 | 0-1 |
| B (bekannt) | 1 | 1 |
| Kontrolle (unbehandelt) | 4 | |

0 = kein Befall, abgestuft bis 5 = Totalbefall

Beispiel C

Blätter von in Töpfen gewachsenen Weizenpflanzen werden mit Sporen des Weizenbraunrostes (Puccinia recondita) künstlich infiziert und 48 Stunden lang bei 20 bis 25°C in einer wasserdampfgesättigten Kammer aufgestellt. Danach werden die Pflanzen mit wäßrigen Spritzbrühen, die in dem Wasser gelöst oder emulgiert eine Mischung aus 80 Gew.% des zu prüfenden Wirkstoffes und 20 Gew.% Natriumligninsulfonat enthalten, besprüht und im Gewächshaus bei Temperaturen zwischen 20 und 22°C und bei 70 bis 80 % relativer Luftfeuchtigkeit aufgestellt. Nach 10 Tagen wird das Ausmaß der Rostpilzentwicklung beurteilt.

| Wirkstoff-Nr. | Befall der Blätter nach Spritzungen mit x %-iger Wirkstoffbrühe x = 0,025 | 0,05 |
|---|---|---|
| 30 | 0 | 0 |
| 31 | 0 | 0 |
| 33 | 0 | 0 |
| 45 | 2 | 0 |
| 46 | 2 | 0 |
| 47 | 0 | 0 |
| 48 | 0 | 0 |
| 56 | 2 | 0 |
| 57 | 2 | 0 |
| 66 | 0 | 0 |
| 75 | 3 | 0 |
| 76 | 2 | 0 |
| 88 | 1 | 0 |
| 89 | 2 | 0 |
| 101 | 0 | 0 |
| 102 | 0 | 0 |
| 104 | 2 | 0 |
| 105 | 0 | 0 |
| 106 | 0 | 0 |
| A (bekannt) | 3 | 3 |
| B (bekannt) | 4 | 4 |
| Kontrolle (unbehandelt) | 4 | |

0 = kein Befall, abgestuft bis 5 = Totalbefall

## Patentansprüche

1. 3-(p-Isopropyl-phenyl)-2-methyl-n-propyl-amine der Formel I

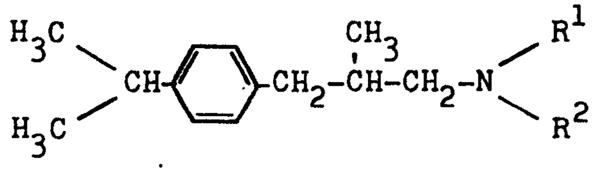

I

in der

$R^1$ Wasserstoff oder einen unverzweigten oder verzweigten Alkylrest mit 1 bis 4 Kohlenstoffatomen, einen unverzweigten oder verzweigten Alkoxyalkylrest mit 2 bis 6 Kohlenstoffatomen, einen gegebenenfalls einfach oder mehrfach durch unverzweigtes oder verzweigtes Alkyl mit 1 bis 4 Kohlenstoffatomen substituierten Cycloalkylrest mit 5 oder 6 Kohlenstoffatomen oder den Formylrest

und

$R^2$ Wasserstoff, einen unverzweigten oder verzweigten Alkylrest mit 1 bis 8 Kohlenstoffatomen, der gegebenenfalls durch Phenyl, das unverzweigte oder verzweigte Alkylgruppen mit 1 bis 4 Kohlenstoffatomen tragen kann, substituiert sein kann, einen unverzweigten oder verzweigten Alkoxyalkylrest mit 2 bis 6 Kohlenstoffatomen, einen Mono- oder Bicycloalkylrest mit 5 bis 9 Kohlenstoffatomen, der einfach oder mehrfach durch unverzweigtes oder verzweigtes Alkyl mit 1 bis 4 Kohllenstoffatomen substituiert sein kann, einen Indanyl-(1)-rest oder einen gegebenenfalls in 1-Stellung durch Methyl substituierte Piperidylrest bedeuten,

und

$R^1$ und $R^2$ zusammen mit dem Stickstoffatom, dessen Substituenten sie sind, einen gesättigten mono- oder bicyclischen Ring mit 5 bis 10 Ringgliedern, der ein weiteres Heteroatom als Ringlied enthalten und einfach oder mehrfach durch unverzweigtes oder verzweigtes Alkyl mit 1 bis 4 Kohlenstoffatomen, Hydroxyl, Hydroxylalkyl mit 1 bis 4 Kohlenstoffatomen, Alkoxy mit 1 bis 4 Kohlenstoffatomen, Pyridyl oder Benzyl substituiert sein kann, einen Pyrolidonrest, einen Maleinimidrest, einen 1-Oxo-iso-indolinrest oder einen 1.2.3.4-Tetrahydrochinolinrest bilden,
oder ihre Salze, Molekül- und Additionsverbindungen.

2. 4-[3'-(p-Isopropylphenyl)-2'-methyl-n-propyl]-2,6-dimethyl-morpholin.

3. 1-[3'-(p-Isopropylphenyl)-2'-methyl-n-propyl]-piperidin.

4. Fungizid, enthaltend einen festen oder flüssigen Trägerstoff und ein Amin gemäß Anspruch 1.

5. Verfahren zur Herstellung eines Amins der Formel I, dadurch gekennzeichnet, daß man 3-(4-Isopropyl-phenyl)-2-methyl-propanal mit einem Amin der Formel II

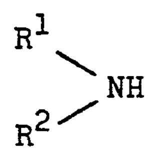

II

in der
$R^1$ und $R^2$ die obengenannten Bedeutungen haben,
in Gegenwart eines Reduktionsmittels bei einer Temperatur im Bereich von 20 bis 250°C umsetzt.

6. Verfahren zur Bekämpfung von Pilzen, dadurch gekennzeichnet, daß man die vor Pilzbefall zu schützenden Pflanzen oder ihre Samen mit einem Amin der Formel I behandelt.

0006992

Nummer der Anmeldung

79101635.5

Europäisches Patentamt

# EUROPÄISCHER RECHERCHENBERICHT

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | DE-A 2 137 275 (BASF)<br>* S.5, Beispiel 1* | 1-3,5 | C 07 D 211/10<br>C 07 D 211/20<br>C 07 D 265/30<br>C 07 D 295/02<br>C 07 C 87/02<br>A 01 N 9/20<br>A 01 N 9/22 |
| A | DE-A- 2 112 249 (LILLY)<br>*Patentansprüche *<br>-- | 1-3,5 | |
| A | DE-A- 2 752 096 (HOFFMANN-LA ROCHE 01-06-1978)<br>*Patentansprüche*<br>-- | 1-6 | |
| A | DE-A- 1 717 104 (CIBA-GEIGY)<br>*Seiten 7 und 8*<br>-- | 4 | RECHERCHIERTE SACHGEBIETE (Int. Cl.3) |
| A | AT-B- 251 559 (CHINOIN)<br>*Gesamtes Dokument*<br>-- | 1-3 | A 01 N 9/00<br>C 07 C 87/00<br>C 07 D 211/10<br>C 07 D 21/20<br>C 07 D 215/06<br>C 07 D 265/30<br>C 07 D 295/02 |
| A | DE-C- 893 341 (TROPONWERKE)<br>*Patentanspruch*<br>-- | 1-3,5 | |
| A | US-A- 2 789 138 (HEINZELMAN)<br>*Spalte 1*<br>-- | 1-3 | |
| D | DE-B- 1 164 152 (BASF)<br>*Gesamtes Dokument"<br>-- | 4 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| D | DE-B- 1 173 722 (BASF)<br>*Gesamtes Dokument"<br>---- | 4 | &: Mitglied der gleichen Patentfamilie, übereinstimmendes Dokument |

X Der vorliegende Recherchenbericht wurde für alle Patentansprüche erstellt.

| Recherchenort | Abschlußdatum der Recherche | Prüfer |
|---|---|---|
| WIEN | 20-08-1979 | HOCHHAUSER |